# EUROPEAN PATENT APPLICATION

(11) **EP 4 782 833 A1**
(43) Date of publication of application: **29.07.2026**
(21) Application number: 24867813.8
(22) Date of filing: 01.05.2024
(51) Int. Cl.: G01N 33/48, G01N 21/27, G06T 7/00, G06T 7/90

(54) **STAINING INTENSITY ACQUISITION METHOD, PROGRAM, AND STAINING INTENSITY ACQUISITION DEVICE**

(30) Priority: 20.09.2023 JP 2023152127
(71) Applicant: SCREEN Holdings Co., Ltd., Kyoto-shi, Kyoto 602-8585 (JP)
(72) Inventor: OGI, Hiroshi, Kyoto-shi, Kyoto 602-8585 (JP); HIRAI, Saya, Kyoto-shi, Kyoto 602-8585 (JP); FURUTA, Tomoyasu, Kyoto-shi, Kyoto 602-8585 (JP)
(74) Representative: Goddar, Heinz J.
(86) International application number: PCT/JP2024/016803
(87) International publication number: WO 2025/062724

(57) **Abstract**

A plurality of high-intensity pixels (422) with relatively high pixel staining intensities are identified in a target region (42) that corresponds to at least part of a cell, the pixel staining intensities being staining intensities derived from pixel values. Then, an average value of the pixel staining intensities of the high-intensity pixels (422) is acquired as a region staining intensity that is a staining intensity in the target region (42). Accordingly, it is possible to acquire an appropriate region staining intensity while avoiding false negatives and false positives in staining assessment.

## Description

### TECHNICAL FIELD

The present invention relates to a technique for acquiring, from a cell image that indicates a plurality of cells stained, a staining intensity in a region corresponding to at least part of each cell.

### CROSS-REFERENCE TO RELATED APPLICATIONS

This application claims priority benefit of Japanese Patent Application No. JP2023-152127 filed in the Japan Patent Office on September 20, 2023, the entire disclosure of which is incorporated herein.

### BACKGROUND ART

In recent year's research in medical or biological sciences, biological specimens such as tissue specimens or cell specimens are analyzed in units of single cells for the purpose of the elucidation of disease mechanisms, biological mechanisms, drug action mechanisms or the like. In immunostaining analysis, for example, quantitative and qualitative analysis is conducted by staining biological products such as protein in various ways and measuring the staining conditions of the biological products.

According to Japanese Patent Application Laid-Open No. 2022-123488 (Document 1), a cell region that corresponds to cells or central or outer peripheral regions of cells is extracted from an image of a stained tissue, and an average concentration within the entire extracted region or a maximum concentration within that region is acquired as a "staining concentration". Then, if the staining concentration is greater than or equal to a predetermined threshold value, the cells are assessed as "positive" about the staining, and if the staining concentration is lower than or equal to the threshold value, the cells are assessed as "negative" about the staining. Being assessed as "positive" means, for example, that the cells contain a significant amount of a specific protein or that an intracellular organelle exists.

In the case where each region extracted from a cell image is assessed as either positive or negative about staining, based on an average of staining intensities (staining concentrations in Document 1) in the region as in Document 1, some regions may be assessed as false-positive if they generally exhibit a relative high staining intensity, or some regions may be assessed as false-negative because they have only small areas with high staining intensities. Besides, if the assessment is made based on only the maximum concentration in the extracted region, pixel values will be strongly affected by abnormal pixels, making it difficult to ensure sufficient accuracy of assessment.

### SUMMARY OF THE INVENTION

It is an object of the present invention to acquire, from a cell image that indicates a plurality of cells stained, an appropriate staining intensity in a target region that corresponds to at least part of each cell.

Aspect 1 of the present invention is a staining-intensity acquisition method of acquiring, from a cell image that indicates a plurality of cells stained, a region staining intensity that is a staining intensity in a target region that corresponds to at least part of each cell including a) identifying a plurality of high-intensity pixels with relatively high pixel staining intensities in the target region, the pixel staining intensities being staining intensities derived from pixel values, and b) acquiring the region staining intensity in the target region, based on pixel values of the plurality of high-intensity pixels.

According to Aspect 1 of the present invention, it is possible to acquire an appropriate staining intensity in the target region.

Aspect 2 of the present invention is the staining-intensity acquisition method according to Aspect 1, in which the plurality of high-intensity pixels are a predetermined number of pixels with a highest pixel staining intensity in the target region.

Aspect 3 of the present invention is the staining-intensity acquisition method according to Aspect 2, in which the predetermined number is proportional to the number of pixels included in the target region.

Aspect 4 of the present invention is the staining-intensity acquisition method according to Aspect 1 (or according to any one of Aspects 1 to 3), in which the plurality of high-intensity pixels are spaced from each other by a predetermined distance or more.

Aspect 5 of the present invention is the staining-intensity acquisition method according to Aspect 1, in which the operation a) includes identifying at least one peak pixel with a higher pixel staining intensity than surrounding pixels in the target region, and identifying, as the plurality of high-intensity pixels, pixels within a circular region of a predetermined size centered on the at least one peak pixel.

Aspect 6 of the present invention is the staining-intensity acquisition method according to Aspect 1 (or according to any one of Aspects 1 to 5), in which the cell image indicates a tissue specimen.

Aspect 7 of the present invention is the staining-intensity acquisition method according to any one of Aspects 1 to 6, in which the target region is extracted as a region representing one entire cell from the cell image.

Aspect 8 of the present invention is the staining-intensity acquisition method according to any one of Aspects 1 to 6 that further includes, before the operation a), c) extracting a provisional target region from the cell image, the provisional target region corresponding at least part of the each cell, and d) dividing the provisional target region into an interior region and an outer peripheral region. The target region is either the interior region or the outer peripheral region.

Aspect 9 of the present invention is a program for causing a computer to acquire, from a cell image that indicates a plurality of cells stained, a region staining intensity that is a staining intensity in a target region that corresponds to at least part of each cell. Executing the program by the computer causes the computer to execute a) identifying a plurality of high-intensity pixels with relatively high pixel staining intensities in the target region, the pixel staining intensities being staining intensities derived from pixel values, and b) acquiring the region staining intensity in the target region, based on pixel values of the plurality of high-intensity pixels.

Aspect 10 of the present invention is a staining-intensity acquisition apparatus of acquiring, from a cell image that indicates a plurality of cells stained, a region staining intensity that is a staining intensity in a target region that corresponds to at least part of each cell including a pixel identifier that identifies a plurality of high-intensity pixels with relatively high pixel staining intensities in the target region, the pixel staining intensities being staining intensities derived from pixel values, and an intensity acquirer that acquires the region staining intensity in the target region, based on pixel values of the plurality of high-intensity pixels.

These and other objects, features, aspects and advantages of the present invention will become more apparent from the following detailed description of the present invention when taken in conjunction with the accompanying drawings.

### BRIEF DESCRIPTION OF DRAWINGS

Fig. 1 is a diagram showing a configuration of a computer that functions as a staining-intensity acquisition apparatus.
Fig. 2 is a block diagram showing a functional configuration of the staining-intensity acquisition apparatus.
Fig. 3 is a flowchart for operations of the computer.
Fig. 4 is a diagram showing a cell image.
Fig. 5 is a diagram showing a target region.
Fig. 6 is a diagram showing positions of high-intensity pixels identified in the target region.
Fig. 7 is a diagram showing a cell image indicating the result of assessment.
Fig. 8 is a diagram showing a state in which the entire target region is stained non-specifically.
Fig. 9 is a diagram for describing another operation example of a staining-intensity acquirer.
Fig. 10 is a diagram for describing another operation example of the staining-intensity acquirer.
Fig. 11 is a flowchart for yet another operation example of the staining-intensity acquirer.
Fig. 12 is a diagram for describing yet another operation example of the staining-intensity acquirer.
Fig. 13 is a flowchart for another operation example of a target-region acquirer.
Fig. 14 is a diagram for describing another operation example of the target-region acquirer.
Fig. 15 is a diagram for describing another operation example of the target-region acquirer.

### DESCRIPTION OF EMBODIMENTS

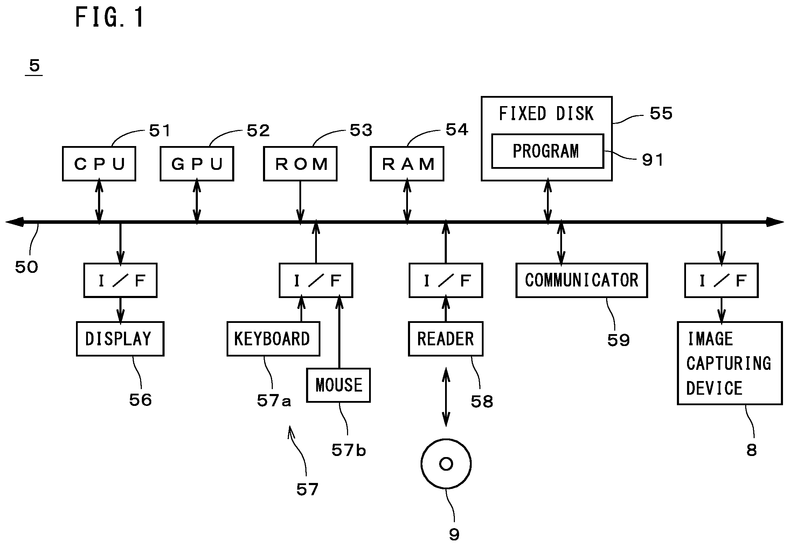

Fig. 1 is a diagram showing a configuration of a computer 5 that functions as a staining-intensity acquisition apparatus according to one embodiment of the present invention. The staining-intensity acquisition apparatus is an apparatus that acquires, from a cell image, a staining intensity in a target region that corresponds to at least part of each of cells (the staining intensity is hereinafter referred to as a "region staining intensity" in order to distinguish it from a staining intensity of each of pixels described later). The cell image is a digital image that indicates a plurality of cells obtained by staining a biological product such as a specific protein or RNA (ribonucleic acid), and is, for example, an image obtained by capturing an image of a biological specimen acquired by immunostaining. The cell image may also be one of a plurality of images obtained by every staining step in multi-immunostaining in which staining and decolorization are repeated.

The computer 5 has a configuration of a general computer system that includes a CPU 51, a GPU 52, ROM 53, RAM 54, a fixed disk 55, a display 56, an input device 57, a reader 58, a communicator 59, and a bus 50. The CPU 51 performs various computational processing. The GPU 52 performs various computation processing related to image processing at high speed. The ROM 53 stores basic programs. The RAM 54 stores various types of information. The fixed disk 55 stores information. The display 56 is a display device that displays various types of information such as images.

The input device 57 includes a keyboard 57a and a mouse 57b that accept input from an operator. The reader 58 reads information from a computer-readable recording medium 9 such as an optical disk, a magnetic disk, a magneto-optical disk, or a memory card. The display 56, the keyboard 57a, the mouse 57b, and the reader 58 are connected to the bus 50 via interfaces (I/Fs). The communicator 59 transmits and receives signals to and from devices or the like located outside the computer 5.

The bus 50 serves as a signal circuit that connects the CPU 51, the GPU 52, the ROM 53, the RAM 54, the fixed disk 55, the display 56, the input device 57, the reader 58, and the communicator 59. The bus 50 is connected to an image capturing device 8 located outside the computer 5 via an I/F. The image capturing device 8 acquires an image of a specimen as a cell image and stores data about the cell image in the fixed disk 55.

In the computer 5, a program 91 is read from the recording medium 9 via the reader 58 and stored in the fixed disk 55 in advance. The program 91 may be stored in the fixed disk 55 via a network. The CPU 51 and the GPU 52 execute computational processing in accordance with the program 91 while using the RAM 54 and the fixed disk 55. The CPU 51 and the GPU 52 function as operation parts in the staining-intensity acquisition apparatus 1. Any other configuration that functions as an operation part may be adopted in addition to the CPU 51 and the GPU 52.

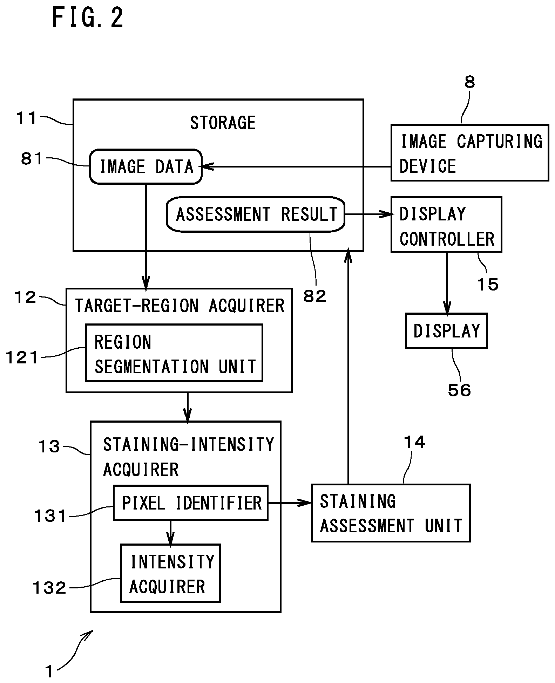

Fig. 2 is a block diagram showing a functional configuration of the staining-intensity acquisition apparatus 1 realized by the aforementioned computer 5 executing computational processing or the like in accordance with the program 91. Fig. 2 also shows a configuration other than the functions of the staining-intensity acquisition apparatus 1. In Fig. 2, a staining-intensity acquirer 13 represents a principal functional configuration of the staining-intensity acquisition apparatus 1. A storage 11 and a target-region acquirer 12 may also be included in the functional configuration of the staining-intensity acquisition apparatus 1. By further realizing a functional configuration of a staining assessment unit 14, the computer 5 serves as an apparatus that assesses whether each cell in a cell image is positive or negative about staining.

The whole or a part of the functional configuration may be realized by a dedicated electric circuit. Alternatively, the functional configuration may be realized by multiple computers. In the functional configuration shown in Fig. 2, the storage 11 is primarily realized by the RAM 54 and the fixed disk 55. The target-region acquirer 12, the staining-intensity acquirer 13, the staining assessment unit 14, and the display controller 15 are realized by the CPU 51, the GPU 52, the ROM 53, the RAM 54, the fixed disk 55, and their surrounding configuration.

The storage 11 stores a cell image that is an image of a biological specimen (hereinafter, also simply referred to as a "specimen") acquired by the image capturing device 8. To be precise, the image to be stored is "image data 81" as shown in Fig. 2, and in the following description, processing to be performed on the image corresponds to processing to be performed on the image data 81.

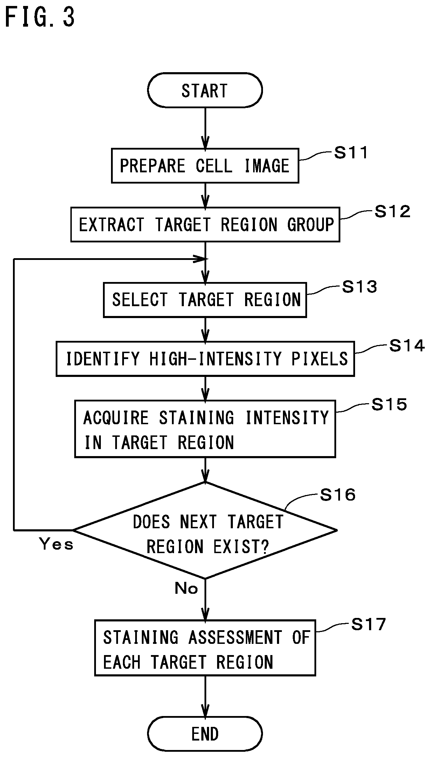

Fig. 3 is a flowchart for operations of the computer 5 and shows an operation of acquiring a region staining intensity that is a staining intensity in each of target regions in a cell image (which may be regarded as a staining intensity of each cell) and assessing whether the target region is positive or negative about staining.

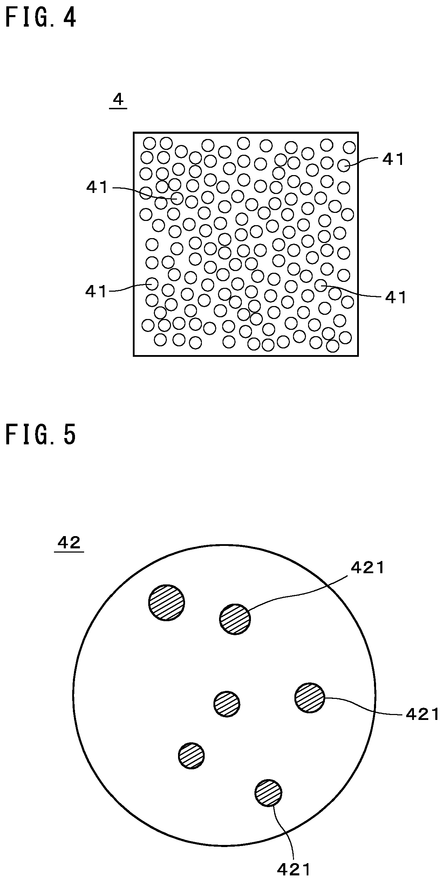

Through the control of the image capturing device 8 by the computer 5, or through the operation of the image capturing device 8, firstly, an image of a specimen is captured and a cell image is prepared in the storage 11 (step S11). As illustrated in Fig. 4, a cell image 4 stored in the storage 11 indicates a plurality of cells 41. In Fig. 4, each cell 41 is simply indicated by a circle.

Then, the target-region acquirer 12 acquires a region that corresponds to at least part of each cell 41 as a target region from the cell image 4 (step S12). In the case where the cell image 4 to be processed is one of a plurality of images obtained by every staining step in multi-immunostaining in which staining and decolorization are repeated, a region that corresponds to the target region may be acquired from a cell image obtained by a different staining step from the cell image 4 (i.e., a different stained image of the same specimen), and this region may be adopted as the target region in the cell image 4. Note that the function of a region segmentation unit 121 in the target-region acquirer 12 will be described later. Hereinafter, a plurality of target regions acquired by the target-region acquirer 12 are referred to as a "target region group".

One target region may be equivalent to a so-called "cell region" that corresponds to one entire cell 41, or may be equivalent to part of a cell region such as a region corresponding to the nucleus of each cell. Various known methods can be adopted for the acquisition of the target regions. Examples of the methods include various known methods of acquiring a cell region, various known methods of acquiring a nuclear region, and known methods of acquiring a region corresponding to at least part of a cell that is not confined to a cellular structure. In the case where single-cell analysis is conducted, target regions and cells are preferably in a one-to-one correspondence with each other. That is, target regions are at least part of cell regions, and cell regions and target regions are in a one-to-one correspondence with each other. In this way, a target region is a region that corresponds to at least part of each cell. The computer 5 does not necessarily have to be have the function of extracting a cell region as long as it has the function of extracting a target region.

Then, the staining-intensity acquirer 13 selects the first target region to be processed (step S13). Fig. 5 is a diagram showing, for example, a target region 42 when the target region 42 corresponds to one cell region. In the target region 42, stained regions 421 exist. In Fig. 5, the regions 421 are shown by circles with parallel diagonal lines in order to facilitate understanding. However, in actuality, the regions 421 are of various shapes with light and shade, and some of them may have unclear contours.

The staining-intensity acquirer 13 includes a pixel identifier 131 and an intensity acquirer 132. The pixel identifier 131 identifies, from pixels within the target region 42, a plurality of pixels (hereinafter, referred to as "high-intensity pixels") with relatively high staining intensities (hereinafter, referred to as "pixel staining intensities") derived from pixel values (step S14). The term "staining intensity" used in the terms "region staining intensity" and "pixel staining intensity" refers to the intensity of staining observed in the cell image. For example, in the case where intensely stained regions appear dark in an image of staining-derived color development captured by a bright-field imaging system, a darker color indicates a higher staining intensity. In the case where intensely stained regions appear bright in an image of light emission caused by fluorochrome staining, a brighter color indicates a higher staining intensity.

Thus, in the case where the cell image is a monochrome image, a larger pixel value may indicate a higher pixel staining intensity, or may indicate a lower pixel staining intensity. In the case where the cell image is a color image, the higher the value of a specific color element of a pixel or the higher the value computed from multiple color elements, the higher or the lower the pixel staining intensity. Note that in the case where the cell image is a color image, an image that has beforehand undergone processing for extracting only expected staining color tone components, such as color separation or color extraction, may be treated as a new cell image to be processed. Similarly, in the case where the cell image is a monochrome image, an image that has undergone image processing may be treated as a new cell image. As will be described later, the same also applies to the relationship between the degree of the region staining intensity derived from the pixel values of a plurality of high-intensity pixels and the brightness or color tone of the target region 42 in the image.

To be expressed generally, the "staining intensity" is a value derived from the pixel values of one or a plurality of pixels included in a region (the pixel staining intensity may be the pixel value itself), and is a value that indicates the intensity of staining. To be more precise, the "pixel staining intensity" is a value based on the pixel value of one pixel, and the "region staining intensity" is a value based on the pixel values of a plurality of high-intensity pixels included in the target region 42.

As described above, as the pixel staining intensity increases, the pixel value will simply increase or decrease (including the case where the value of one color component of a color pixel value or the value derived from multiple color components corresponds to the pixel value). Thus, without actually obtaining the pixel staining intensities, the pixel identifier 131 of the staining-intensity acquirer 13 can identify a plurality of high-intensity pixels with relatively high pixel staining intensities in the target region 42. For example, the pixel identifier 131 identifies, as high-intensity pixels, a predetermined number of pixels from among pixels with the highest pixel value or from among the pixels with the lowest pixel value. Accordingly, a predetermined number of pixels that exhibit the highest pixel staining intensity in the target region 42 can be identified easily as a plurality of high-intensity pixels. Note that the "predetermined number of pixels that exhibit the highest pixel staining intensity" refer to top-ranked pixels within a predetermined number when pixels are sorted in descending order of the pixel staining intensity.

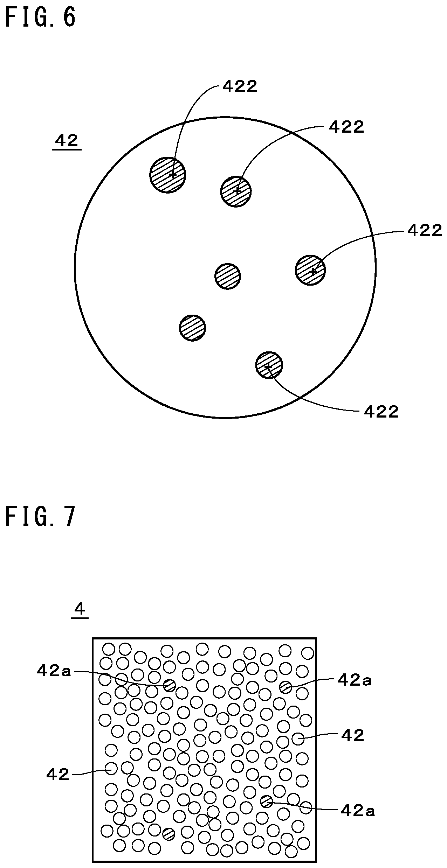

Fig. 6 is a diagram showing an example in which the positions of four high-intensity pixels 422 identified in the target region 42 are indicated by +. There are no particular limitations on the number of high-intensity pixels 422 to be identified in each target region 42. However, in a preferable example, the number of high-intensity pixels 422 is proportional to the number of pixels included in each target region 42 in one image. In another preferable example, the number of high-intensity pixels 422 remains constant, independently from the size of the target region 42. The number of high-intensity pixels 422, or the ratio of the number of high-intensity pixels 422 to the number of pixels in the target region 42 is set as appropriate depending on the actual size of the target region 42, the resolution of the cell image, and staining characteristics (e.g., the amount of a target protein or intracellular organelle that is present, the size of a target substance, ease of staining).

The ratio of the number of high-intensity pixels 422 to the number of pixels in the target region 42 may be determined automatically by learning. For example, for variously stained cell images, a computer learning machine may be trained to recognize the positive or negative status of numerous target regions 42 and optimum combinations of the number of high-intensity pixels 422. Then, when an input image is input to the learning machine, the learning machine may output the ratio of the number of high-intensity pixels 422 to the number of pixels in the target region 42.

Then, the intensity acquirer 132 acquires an average value of the pixel staining intensities at the identified high-intensity pixels 422 as the region staining intensity in the target region 42 (step S15). As described previously, the pixel values may be used, instead of the pixel staining intensities. Thus, an average value of the pixel values of the high-intensity pixels 422 may be acquired as the region staining intensity in step S15. Of course, the pixel staining intensity of each high-intensity pixel 422 may be obtained by computation, and an average value of the pixel staining intensities may be acquired as the region staining intensity in the target region 42. In this way, the intensity acquirer 132 acquires the region staining intensity in the target region 42, based on the pixel values of the high-intensity pixels 422. In the case where the number of high-intensity pixels 422 is proportional to the number of pixels included in the target region 42, the region staining intensity can be obtained while reducing the influence of the size of the target region 42.

When the region staining intensity is acquired for the one target region 42, the staining-intensity acquirer 13 checks whether the target region group includes the next target region 42 whose region staining intensity has not yet been obtained (step S16), and if there is any, the next target region 42 is selected to acquire the region staining intensity (steps S13 to S15). When the region staining intensity has been acquired for all of the target regions 42 (step S16), the procedure proceeds to processing that is performed by the staining assessment unit 14.

The staining assessment unit 14 has a threshold value that is set in advance via the input device 57 by an operator and compares the region staining intensity in each target region 42 of the target region group with the threshold value. If the region staining intensity in the target region 42 is higher than the threshold value, the target region 42 is assessed as positive about staining (step S17). Note that in the case where a lower average pixel value of the high-intensity pixels included in the target region 42 indicates a higher region staining intensity and the staining assessment unit 14 directly compares the average pixel value with the threshold value, the target region 42 is assessed as positive about staining when the average pixel value is smaller than the threshold value. An assessment result 82 for each target region 42 is stored in the storage 11.

The assessment result 82 is displayed on the display 56 under the control of the display controller 15. For example, the cell image 4 is displayed on the display 56, with coloring given to positive target regions 42a among the target regions 42 in the cell image 4 as shown in Fig. 7.

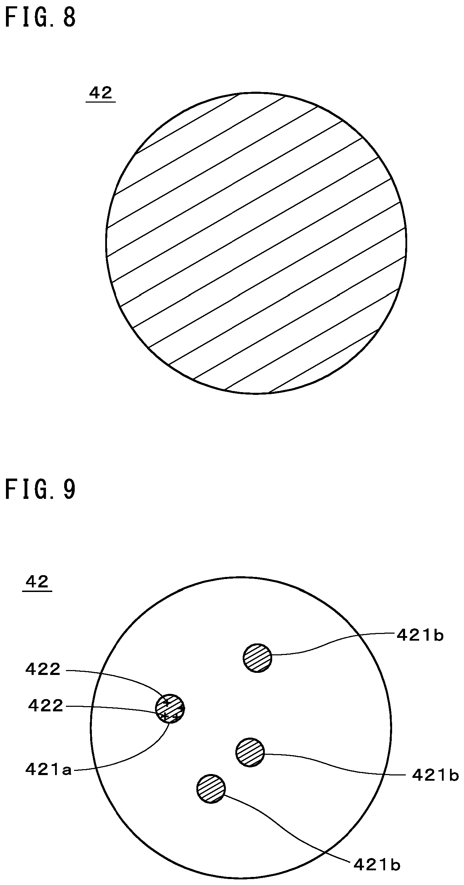

In the staining-intensity acquisition apparatus 1, staining assessment, i.e., assessment as to whether or not each target region is positive about staining, can be properly conducted by using the pixel staining intensities of the high-intensity pixels 422. For example, the entire target region 42 may be stained non-specifically as shown in Fig. 8 and exhibit about 60% of the highest staining intensity. In the case of such a staining condition, if an average value of the pixel staining intensities in the entire target region 42 is acquired as the region staining intensity in the target region 42 as in the conventional technique, the target region 42 will be mis-assessed as positive with a threshold value of 50%, even if the target region 42 should not be assessed as positive. On the other hand, in the case shown in Fig. 6, the target region 42 has only small areas with high staining intensities, and if an average of the pixel staining intensities in the entire target region 42 is obtained as the region staining intensity in the target region 42 and this value is for example 40%, the target region 42 will be mis-assessed as negative, even though the target region 42 should be assessed as positive.

In contrast, in the case of using the pixel staining intensities of the high-intensity pixels 422, if the average of the pixel staining intensities of the high-intensity pixels 422 is obtained as the region staining intensity, the region staining intensity acquired is, for example, 80% and the target region 42 is assessed as positive. In this way, by using the pixel staining intensities of the high-intensity pixels 422 (i.e., by using the pixel values), it is possible to appropriately receive a positive result from the assessment when the target region has an intensely stained area, while avoiding false positives or false negatives in the assessment.

The staining assessment using the high-intensity pixels 422 is particularly suitable for cases where staining assessment is conducted on cell images that have undergone nuclear staining or cytoplasmic staining in which only part of the target region 42 is stained intensely. Besides, the staining assessment can be conducted appropriately even if there exists noise called background noise, where part of or the entire cellular structure is colored faintly and non-specifically. Background noise occurs easily during staining of tissue specimens than cultured cells, so that the staining assessment using the high-intensity pixels 422 are suitable for assessing the staining of tissue specimens.

The staining assessment using the high-intensity pixels 422 is also suitable for assessing the staining of proteins with high intracellular localization or uneven distribution in the presence of background noise. For example, the staining assessment using the high-intensity pixels is more suitable for assessing the staining of fibrillin localized in nucleoli than the staining of actin forming the cytoskeleton. Moreover, the staining assessment using the high-intensity pixels is also suitable for assessing the staining of images with low S/N ratios, and is for example more suitable for staining assessment using the enzyme antibody technique with a color coupler and a bright-field optical system than for staining assessment using fluorochrome and a fluorescence optical system.

Note that all functions and effects in the case of obtaining the region staining intensity using the above-described high-intensity pixels 422 apply equally in the case of obtaining the region staining intensity through identification of high-intensity pixels using peak pixels, which will be described later.

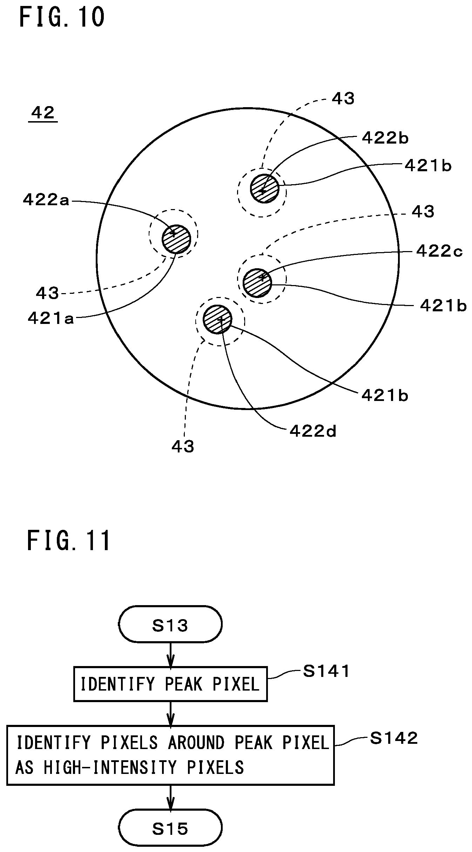

Figs. 9 and 10 are diagrams for describing another operation example of the staining-intensity acquirer 13. Fig. 9 is a diagram showing an example of the target region 42 that should be assessed as negative. In the target region 42 shown in Fig. 9, there exist one intensely stained region 421a and multiple faintly stained small regions 421b that should not be considered stained. In the case of identifying the high-intensity pixels 422 in the example shown in Fig. 9, if a predetermined number of pixels with high staining intensities are extracted from the region 421a, an average value of the pixel staining intensities of the high-intensity pixels 422 becomes high, and the target region 42 will be mis-assessed as positive.

In view of this, the improved staining intensity acquirer 13 acquires, as the high-intensity pixels 422, pixels that are spaced from each other by a predetermined distance or more. Specifically, as shown in Fig. 10, firstly, a pixel 422a with the highest pixel staining intensity is identified as a high-intensity pixel 422. Then, from among pixels that are spaced from this pixel 422a at a distance greater than or equal to the radius of a circle indicated by a broken line 43, a pixel 422b with the highest pixel staining intensity is identified as the next high-intensity pixel 422. Then, from among pixels that are spaced from all the already-identified high-intensity pixels 422a and 422b at a distance greater than or equal to the radius of the circle indicated by the broken line 43, a pixel 422c with the highest pixel staining intensity is identified as the next high-intensity pixel 422. The above-described operation is repeated to identify a predetermined number of high-intensity pixels 422 (pixels 422a to 422d shown in Fig. 10).

Since the pixel staining intensities of the pixels 422b, 422c, and 422d are not high, the average value of the pixel staining intensities of the high-intensity pixels 422, i.e., the region staining intensity, becomes low. As a result, the target region 42 is correctly assessed as negative. If the high-intensity pixels 422 are further spaced away from one another, it is possible to obtain the region staining intensity while avoiding an increase in the influence of specific regions with high staining intensities.

The distance to be provided between the high-intensity pixels 422, i.e., the radius of the circles indicated by the broken lines 43 in Fig. 10, may be determined automatically. For example, this distance may be determined to be a value that is proportional to an average radius when multiple cell regions are regarded as circles and that is inversely proportional to the square root of the number of high-intensity pixels 422 to be identified. Thus, the "predetermined distance" at which the high-intensity pixels 422 are to be spaced from each other may vary for each target region 42.

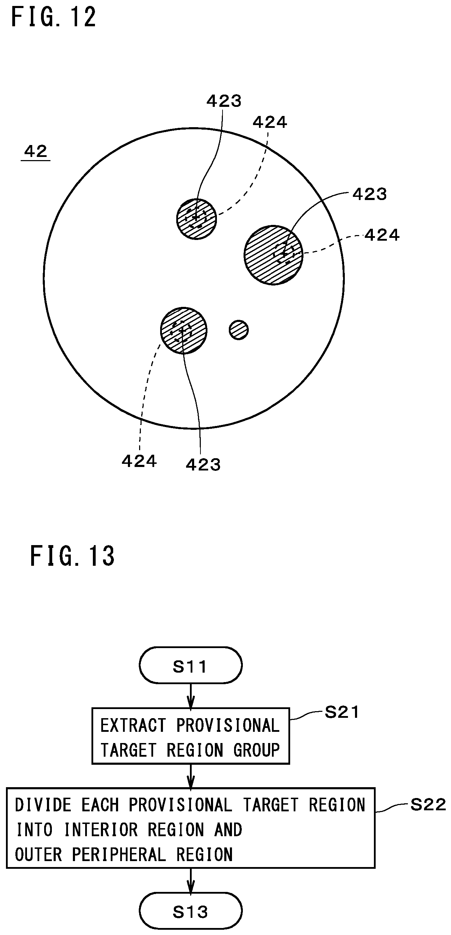

Next, yet another operation example of the staining intensity acquirer 13 shown in Fig. 2 is described with reference to Figs. 11 and 12. In this operation example, the operation shown in Fig. 11 is executed by the pixel identifier 131 in step S14 shown in Fig. 3.

First, the pixel identifier 131 identifies at least one peak pixel 423 in the target region 42 as shown in Fig. 12 (step S141). In the target region 42, the peak pixel 423 is a pixel that exhibits a higher pixel staining intensity than surrounding pixels, i.e., a pixel that is located at a position where the pixel staining intensity peaks when viewed two-dimensionally. It is, however, noted that, in order to exclude peak pixels in regions with low staining intensities, a lower limit is set for the pixel staining intensity required to be identified as peak pixels 423. Moreover, in the case where a pixel exists at a peak position but there is a small number of surrounding pixels forming the peak, such a pixel may be excluded from the peak pixels. The peak pixels are pixels that exhibit pixel staining intensities higher than or equal to a predetermined intensity and that are located at the tops of peaks (of the staining intensities) larger than or equal to the predetermined size.

Then, the pixel identifier 131 identifies pixels within circular regions 424 of a predetermined size centered on at least one peak pixel 423, as a plurality of high-intensity pixels (step S142). All the radii of the circular regions 424 may be the same, or the higher the pixel staining intensity of the peak pixel 423, the larger the radius may be. That is, the "predetermined size" may be the same for all the circular regions 424, or may vary for each circular region 424.

Thereafter, the intensity acquirer 132 acquires an average value of the pixel staining intensities of all the pixels within at least one circular region 424, as the region staining intensity in the target region 42. In the case where the number of circular regions 424 is two or more, after an average value of the pixel staining intensities is obtained for each circular region 424, an average value of these multiple average values may be determined as the region staining intensity in the target region 42. In this way, the intensity acquirer 132 acquires the region staining intensity in the target region 42, based on the pixel staining intensities of the high-intensity pixels within the circular regions 424 of the predetermined size centered on at least one peak pixel 423.

The identification of the peak pixels 423 can be easily accomplished using, for example, a library "OpenCV" for computer vision of open source. By using the peak pixels 423 to acquire the region staining intensity in the target region 42, more accurate staining assessment becomes possible in the case where staining occurs with multiple positive spots scattered within the target region 42. Therefore, the acquisition of the region staining intensity using the peak pixels 423 is suitable for the case where the pixel identifier 131 identifies a plurality of peak pixels 423 within one target region 42. The staining assessment using the peak pixels 423 is particularly suitable for the case where staining assessment is conducted on cell images that have undergone nuclear staining or cytoplasmic staining in which only part of the target region 42 is stained intensely.

Next, another operation example of the target region acquirer 12 shown in Fig. 2 is described with reference to Figs. 13 to 15. In the other operation example, the target region acquirer 12 executes steps S21 and S22 shown in Fig. 13, instead of step S12 shown in Fig. 3. First, the target region acquirer 12 extracts a provisional target region group from the cell image 4 (step S21). One provisional target region may be equivalent to a so-called "cell region" that corresponds to one entire cell, or may be equivalent to part of a cell region such as a region corresponding to the nucleus of a cell. In the case of single-cell analysis, provisional target regions and cells are preferably in a one-to-one correspondence. That is, a provisional target region is at least part of a cell region, and cell regions and provisional target regions are in a one-to-one correspondence. Thus, a provisional target region is a region that corresponds to at least part of each cell. Computer 5 does not necessarily have to have the function of extracting a cell region as long as it has the function of extracting a provisional target region.

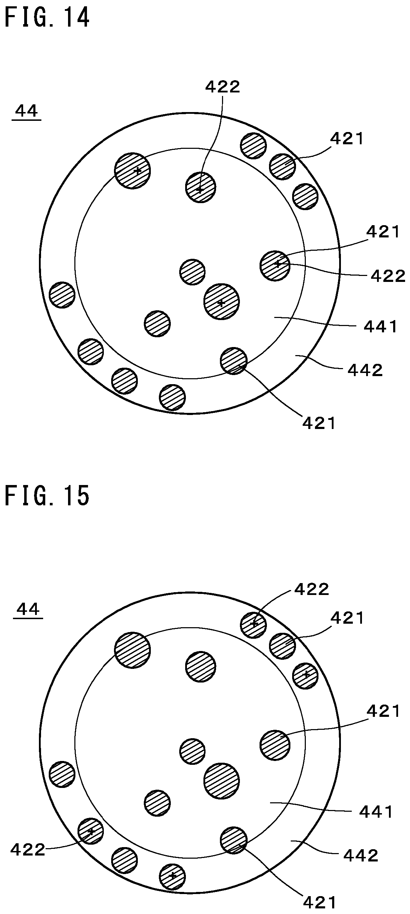

Fig. 14 is diagram showing an example of a provisional target region 44. Regions 421 indicated by circles with parallel diagonal lines represent stained regions, but in actuality, the regions 421 are of various sizes and shapes with light and shade. Some of the regions 421 may have unclear contours. The target region acquirer 12 has the function of the region segmentation unit 121 shown in Fig. 2. The region segmentation unit 121 divides each provisional target region 44 into an interior region 441 on the inner side and an outer peripheral region 442 that corresponds to an outer peripheral portion (step S22). The outer peripheral region 442 is a region of a fixed width along the outer peripheral edge of the provisional target region 44. The interior region 441 is a region located inward of the outer peripheral region 442. The target region acquirer 12 acquires either the interior region 441 or the outer peripheral region 442 as a target region. That is, the interior regions 441 of all the provisional target regions 44 are acquired as target regions, or the outer peripheral regions 442 of all the provisional target regions 44 are acquired as target regions. From then on, steps S13 to S17 shown in Fig. 3 are executed.

Specifically, in the case where the interior region 441 is treated as the target region, among pixels within the interior region 441, those with relatively high pixel staining intensities are identified as the high-intensity pixels 422 in step S14 shown in Fig. 3. In Fig. 14, the high-intensity pixels 422 are indicated by +. On the other hand, in the case where the outer peripheral region 442 is treated as the target region, among pixels within the outer peripheral region 442, those with high pixel staining intensities are identified as the high-intensity pixels 422 in step S14 as shown in Fig. 15. Then, the staining intensity in the target region is acquired (step S15). After the staining intensities in all the target regions have been acquired (step S16), staining assessment is conducted on all the target regions (step S17).

By setting the interior region 441 as the target region, the staining of the interior region 441 can be assessed appropriately even if the outer peripheral region 442 is stained non-specifically. For example, in the case where the nucleus of a cell or a specific protein in the vicinity of the nucleus is required to be stained and detected, it is possible to eliminate the influence of the staining of a cell membrane. It is also possible to eliminate the influence of the staining of adjacent cells. In the case where the outer peripheral region 442 is treated as the target region, the staining of the outer peripheral region 442 can be assessed appropriately even if the interior region 441 is stained non-specifically. For example, in the case where a specific protein in a cell membrane is required to be stained and detected, it is possible to eliminate the influence of staining of areas other than the cell membrane.

Of course, the provisional target region 44 is not limited to a cell region (the region representing the entire cell). For example, the provisional target region 44 may be extracted as a nucleus and its surrounding area, and a region inside the nucleus may be acquired as the interior region 441 and a region in the outer peripheral portion of the nucleus as the outer peripheral region 442. By dividing the provisional target region 44 into the interior region 441 and the outer peripheral region 442 and treating only one of them as a target region, it is possible to effectively suppress artifacts for analysis and to acquire a more robust region staining intensity.

The above-described embodiment may be modified in various ways.

In the example shown in Fig. 6, a predetermined number of pixels with the highest pixel staining intensity in the target region 42 are identified as the high-intensity pixels 422. In the example shown in Fig. 12, at least one peak pixel 423 and its surrounding pixels are identified as the high-intensity pixels. In this way, the high-intensity pixels may be identified by various methods as long as they exhibit relatively high pixel staining intensities. The high-intensity pixels are preferably included in the top 50% (more preferably in the top 30%) of pixels with high pixel staining intensities among all the pixels in the target region 42. That is, the high-intensity pixels may be selected from among the top 50% of pixels with high pixel staining intensities by various methods. Then, various computations are performed on the pixel values of the high-intensity pixels to acquire the region staining intensity in the target region 42.

The procedure for the operations shown in Fig. 3 may be modified in various ways within the scope of similar functions. For example, each time the region staining intensity in one target region 42 is acquired, the region staining intensity may be compared with a threshold value, and the procedure may proceed to the operation of acquiring the region staining intensity in the next target region 42.

The method using either the interior region 441 or the outer peripheral region 442 shown in Figs. 13 to 15 can be applied to the method of acquiring the region staining intensity by using the peak pixels 423 shown in Figs. 11 and 12.

The staining according to the present embodiment is not limited to multiple staining and is also not limited to staining using antibodies. The present embodiment is applicable to staining assessment for a variety of staining as long as biological substances are stained. The present embodiment is preferably suitable for cases where staining produces light and shade in cells. The present embodiment is also suitable for the staining of a specific protein.

The configurations of the above-described preferred embodiment and variations may be appropriately combined as long as there are no mutual inconsistencies.

While the invention has been shown and described in detail, the foregoing description is in all aspects illustrative and not restrictive. It is therefore understood that numerous modifications and variations can be devised without departing from the scope of the invention.

### REFERENCE SIGNS LIST

- 1: staining-intensity acquisition apparatus
- 4: cell image
- 5: computer
- 13: staining-intensity acquirer
- 41: cell
- 42: target region
- 44: provisional target region
- 91: program
- 131: pixel identifier
- 132: intensity acquirer
- 422: high-intensity pixel
- 423: peak pixel
- 424: circular region (centered on a peak pixel)
- 441: interior region
- 442: outer peripheral region
- S14, S15, S21, S22, S141, S142: step

## Claims

1. A staining-intensity acquisition method of acquiring, from a cell image that indicates a plurality of cells stained, a region staining intensity that is a staining intensity in a target region that corresponds to at least part of each cell, comprising:
a) identifying a plurality of high-intensity pixels with relatively high pixel staining intensities in said target region, the pixel staining intensities being staining intensities derived from pixel values; and
b) acquiring said region staining intensity in said target region, based on pixel values of said plurality of high-intensity pixels.

2. The staining-intensity acquisition method according to claim 1, wherein
said plurality of high-intensity pixels are a predetermined number of pixels with a highest pixel staining intensity in said target region.

3. The staining-intensity acquisition method according to claim 2, wherein
said predetermined number is proportional to the number of pixels included in said target region.

4. The staining-intensity acquisition method according to claim 1, wherein
said plurality of high-intensity pixels are spaced from each other by a predetermined distance or more.

5. The staining-intensity acquisition method according to claim 1, wherein
said operation a) comprises:
identifying at least one peak pixel with a higher pixel staining intensity than surrounding pixels in said target region; and
identifying, as said plurality of high-intensity pixels, pixels within a circular region of a predetermined size centered on said at least one peak pixel.

6. The staining-intensity acquisition method according to claim 1, wherein
said cell image indicates a tissue specimen.

7. The staining-intensity acquisition method according to any one of claims 1 to 6, wherein
said target region is extracted as a region representing one entire cell from said cell image.

8. The staining-intensity acquisition method according to any one of claims 1 to 6, further comprising:
before said operation a),
c) extracting a provisional target region from said cell image, the provisional target region corresponding at least part of said each cell; and
d) dividing said provisional target region into an interior region and an outer peripheral region,
wherein said target region is either said interior region or said outer peripheral region.

9. A program for causing a computer to acquire, from a cell image that indicates a plurality of cells stained, a region staining intensity that is a staining intensity in a target region that corresponds to at least part of each cell,
executing said program by the computer causing said computer to execute:
a) identifying a plurality of high-intensity pixels with relatively high pixel staining intensities in said target region, the pixel staining intensities being staining intensities derived from pixel values; and
b) acquiring said region staining intensity in said target region, based on pixel values of said plurality of high-intensity pixels.

10. A staining-intensity acquisition apparatus of acquiring, from a cell image that indicates a plurality of cells stained, a region staining intensity that is a staining intensity in a target region that corresponds to at least part of each cell, comprising:
a pixel identifier that identifies a plurality of high-intensity pixels with relatively high pixel staining intensities in said target region, the pixel staining intensities being staining intensities derived from pixel values; and
an intensity acquirer that acquires said region staining intensity in said target region, based on pixel values of said plurality of high-intensity pixels.
